# EUROPEAN PATENT SPECIFICATION

(11) **EP 1 841 748 B1**
(45) Date of publication and mention of the grant of the patent: **28.12.2011**
(21) Application number: 05822319.9
(22) Date of filing: 30.12.2005
(51) Int. Cl.: C07D 277/22, C07C 391/02

(54) **ORGANOSELENIUM CONTAINING COMPOUNDS AND THEIR USE**
ORGANOSELENHALTIGE VERBINDUNGEN UND DEREN VERWENDUNG
COMPOSÉS DE TYPE ORGANOSÉLÉNIUM ET APPLICATIONS

(30) Priority: 31.12.2004 KR 20040118109; 30.12.2005 KR 20050135761
(43) Date of publication of application: 10.10.2007
(73) Proprietor: Seoul National University Industry Foundation, Seoul 151-818 (KR)
(72) Inventor: HAM, Jungyeob, Gwanak-gu, Seoul-city 151-770 (KR); KO, Jaeyoung, Seoul-city 151-815 (KR); HWANG, Hoosang, Kunpo-city, Kyeongki-do 435-824 (KR)
(74) Representative: Albrecht, Thomas
(86) International application number: PCT/KR2005/004685
(87) International publication number: WO 2006/071103

(56) References cited:
- WO-A-01/00603
- WO-A1-01/00603
- WO-A1-02/50047
- WO-A1-99/46232
- KR-A- 20030 086 373
- KR-A- 20050 078 836
- DATABASE CA [Online] CHEMICAL ABSTRACTS SERVICE, COLUMBUS, OHIO, US; 1930, KEIMATSU, S. ET AL: "Organic compounds of selenium. I" XP002556971 retrieved from STN Database accession no. 1930:49052 & YAKUGAKU ZASSHI , 50, 531-39;GERMAN ABSTRACT 79-82 CODEN: YKKZAJ; ISSN: 0031-6903, 1930,
- MUELLER, EUGEN ET AL: "Oxygen radicals. XXIV. Investigations of selenium-containing aroxyls by electron resonance" ANNALEN DER CHEMIE, JUSTUS LIEBIGS; CODEN: 9X224Y, vol. 657, 1962, pages 5-12, XP002556970

## Description

### Technical Field

The present invention relates to a novel organoselenium compound, a preparation method thereof and an activator of peroxisome proliferator activated receptor δ (PPARδ) comprising the same.

### Background Art

Of the currently known 48 nuclear receptors, three subtypes of peroxisome proliferator activated receptor (PPARs) are reported. They are PPARα, PPARγ and PPARδ (Nature, 1990, 347, p. 645-650; Proc. Natl. Acad. Sci. USA, 1994, 91, p. 7335-7359). PPARα, PPARγ and PPARδ show different biological functions and have different expression sites. PPARα is mainly expressed in the heart, kidney, skeletal muscle and large intestine (Mol. Pharmacol. 1998, 53, p. 14-22; Toxicol. Lett. 1999, 110, p. 119-127; J. Biol. Chem. 1998, 273, p. 16710-16714) and is related with β-oxidation of peroxisome and mitochondrion (Biol. Cell 1993, 77, p. 67-76; J. Biol. Chem. 1997, 272, p. 27307-27312). PPARγ is weakly expressed in the skeletal muscle, but it is strongly expressed in the fat tissue. It is known to take part in the differentiation of fat cells, storing of energy as fat and control of insulin-glucose homeostasis (Moll. Cell 1999, 4, p. 585-594. p. 597-609. p. 611-617). Whereas PPARα and PPARγ genes show tissue-specific expression patterns, PPARδ is expressed in nearly all tissues, although with different levels (J. Bio. Chem. 1995, 270, p. 2367-2371; Endocrinology 1996, 137, p. 354-366). According to the researches thus far, PPARδ is known to play a key role in the expression of gametes (Genes Dev. 1999, 13, p. 1561-1574.) and be involved in the differentiation of nerve cells in the central nervous system (CNS) (J. Chem. Neuroanat 2000, 19, p. 225-232) and the treatment of wounds through antiphlogistic action (Genes Dev. 2001, 15, p. 3263-3277; Proc. Natl. Acad. Sci. USA 2003, 100, p. 6295-6296). According to a recent research, PPARδ has been proved to be involved in the differentiation of fat cells and the fat metabolism (Proc. Natl. Acad. Sci. USA 2002, 99, p. 303-308; Mol. Cell. Biol. 2000, 20, p. 5119-5128). PPARδ has been proved to activate the expression of the uncoupling proteins (UCPs), which are key genes involved in the β-oxidation of fatty acid and the energy metabolism (Nature 2000, 406, p. 415-418; Cell 2003, 113, p. 159-170; PLoS Biology 2004, 2, p. 1532-1539). Accordingly, the control of UCP through PPARδ may be an effective way of treating obesity.

The action of PPARδ has been discovered not only by the genetic researches but also by the development of specific ligands of PPARδ. GW2433, the first synthesized activation factor of PPARδ, has been proposed as a material capable of treating ateriosclerosis by Glaxo Smith Kline (WO 92/10468). And, L-165041 of Merck showed the effect of reducing the blood level of glucose and triglyceride (TG) (J. Biol. Chem. 1999, 274, p. 6718-6725). A mouse model (db/db) test showed that it may be used to treat obesity and diabetes, since the HDL-cholesterol could be increased to some extent (FEBS Lett. 2000, 473, p. 333-336; WO 97/28115). YM-16638, which was developed by Yamanouchi Pharma of Japan, showed the effect of reducing the blood level of cholesterol and LDL cholesterol (WO 99/04815). GW501516([2-methyl-4-[[[4-methyl-2-[4-(trifluoromethyl)phenyl]-1,3-thiazol-5-yl]methyl]sulfanyl]phenoxy]acetic acid), a selective ligand of PPARδ recently developed by Glaxo Smith Kline, showed much better physiological effect than the preceding ligand. GW501516 showed excellent efficiency in treating obesity in mice (Cell 2003, 113, p. 159-170) and was proved to be effective in treating cardiovascular diseases in primates by effectively increasing high density lipoproteins (HDLs) and reducing low density lipoproteins (LDLs) (Proc. Natl. Acad. USA 2001, 98, p. 5306-5311, 2003, 100, p. 1268-1273). The material and the preparation method thereof are disclosed in the world patent publication and literatures (WO 01/00603; Bioorg. Med. Chem. Lett. 2003, 13, p. 1517-1521; J. Org. Chem. 2003, 68, p. 9116-9118). The GW501516-related compounds of the patents WO 01/00603, WO 02/50048 and WO 02/062774 are limited to those having ether, thioether or alkyl ((CR₁₀R₁₁)ₙ, n = 1 or 2) groups. Recently, there was a report that, although the activation of PPARδ does not induce colon cancer, it can at least promote the proliferation of existing colon cancer cells (Nat. Med. 2004, 10, p. 245-247, p. 481-483). This report suggests that more researches on the relationship between cancer and PPARδ and development of safer compound are required.

Martin L. Smith et al. of Indiana State University reported that selenomethionine, an amino acid containing the nonmetal element selenium (Se), induces the activation of p53, a tumor suppressor gene, and thus reduces the risk of cancer (Proc. Natl. Acad. USA 2002, 99, p. 14548-14553). The antitumor activity of selenium was confirmed through experiments, which showed that the intake of the substance is very important. Selenium is an essential nonmetal trace element belonging to the group VIA along with oxygen (O) and sulfur (S) (Eur. J. Clin. Nutr. 2004, 58, p. 391-402) and is known to protect cells from the free radicals generated by the normal oxygen metabolism as antioxidant (Regul. Toxicol. Pharm. 2003, 38, p. 232-242) and helps the normal function of the immune system and thyroid gland (Pharmacol. Ther. 1998, 79, p. 179-192; Immunol. Today 1998, 19, p. 342-245).

Thus, if selenium is used to develop a ligand showing activity to PARδ, it is highly probable that treatments for cardiovascular diseases, cholesterol depressants, diabetic treatments and treatments for obesity without cancer-related side effects can be developed.

### Disclosure of the Invention

It is an object of the present invention to provide a novel organoselenium compound, a novel ligand having activity to PPARδ, and a preparation method thereof.

It is another object of the present invention to provide a convenient preparation method of the organoselenium compound comprising the steps of protecting the phenol group of a phenol compound with a Grignard reagent without introduction of a special protecting group, performing a reaction with an organometal reagent without a special separation process and subsequently performing a reacting with selenium(Se).

### Best Mode for Carrying Out the Invention

The present invention relates to a novel organoselenium compound represented by the formula I below and a preparation method thereof: 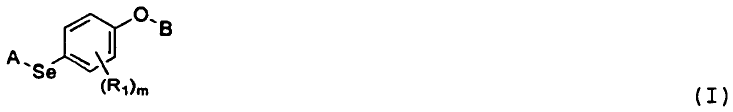 where
A is 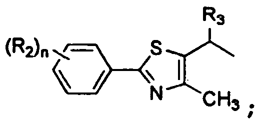 B is hydrogen or 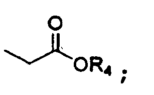 R₁ is independently C₁-C₄ alkyl, C₁-C₄ alkyloxy, C₁-C₄ alkylthioxy, C₁-C₄ alkylamine, fluorine or chlorine;
R₂ is independently C₁-C₄ alkyl, C₁-C₄ alkyl substituted with at least one halogen or halogen;
R₃ is hydrogen, C₁-C₄ alkyl or 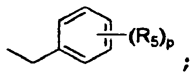 R₄ is hydrogen, alkali metal ion or C₁-C₇ alkyl or aryl;
R₅ is independently C₁-C₄ alkyl, C₁-C₄ alkyl substituted with at least one halogen or halogen;
m is an integer from 0 to 4;
n is an integer from 0 to 5; and
p is an integer from 0 to 5. 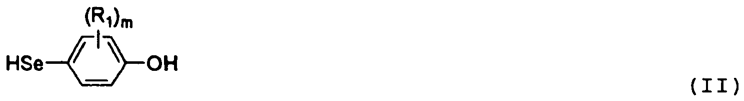 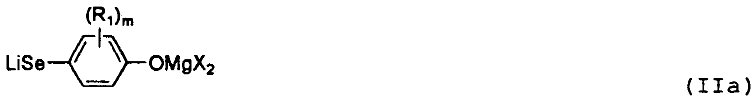 where
R₁ and m are the same as defined in the formula I; and
X₂ is chlorine, bromine or iodine.

The organoselenium compound represented by the formula II or IIa is useful as intermediate for preparing various organoselenium compounds.

The present invention also includes an organoselenium compound represented by the formula III, which is a racemic compound or an optical isomer prepared from the compound represented by the formula II or IIa, and a compound represented by the formula IV, which can be prepared from the compound represented by the formula III: 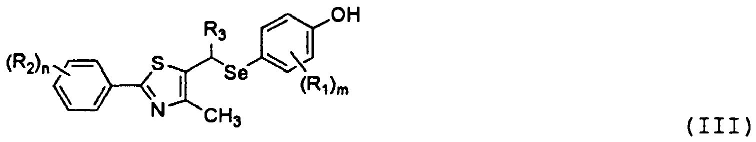 where
R₁ to R₃, m, n and p are the same as defined in the formula I. 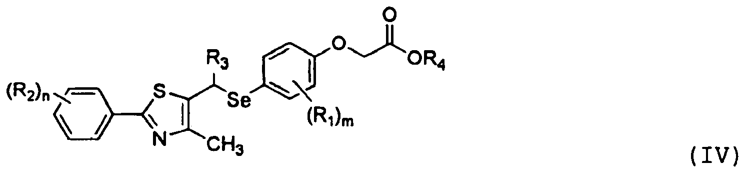 where
R₁ to R₄, m, n and p are the same as defined in the formula I.

The organoselenium compound represented by the formula IV is characterized in that it has an activity for peroxisome proliferator activated receptor δ (PPARδ).

Of the organoselenium compound represented by the formula IV, those where the R₄ is methyl, ethyl, n-propyl, i-propyl, n-butyl, sec-butyl, tert-butyl, phenyl or benzyl group for protecting the carboxylic group and is substituted with C₁-C₇ alkyl or aryl are particularly preferable compounds having an activity for peroxisome proliferator activated receptor δ (PPARδ). Especially, methyl, ethyl or tert-butyl is preferable. The alkali metal ion is Li⁺, Na⁺, K⁺, Ca⁺, etc., preferably hydrogen or Na⁺.

The novel compounds of the present invention can be prepared by the scheme 1 and scheme 2 below.

As seen in the scheme 1, the 4-halogen phenol compound represented by the formula V is prepared into the compound represented by the formula Va after protecting the alcohol group with a Grignard reagent. The halogen group of the compound represented by the formula Va is substituted with lithium and the compound is reacted with selenium to obtain the metal-selenium alcohol intermediate represented by the formula IIa. Subsequently, the compound is reacted with the compound represented by the formula VI (where X₃ is chlorine, bromine, iodine or other leaving group having good reactivity for nucleophilic substitution), without separation or purification, to obtain the selenium ether compound represented by the formula IIIa. The compound is reacted with an alkyl halogen acetate to prepare the compound represented by the formula IVb, which is ester hydrolyzed to obtain the compound represented by the formula IVc with high purity and good yield.

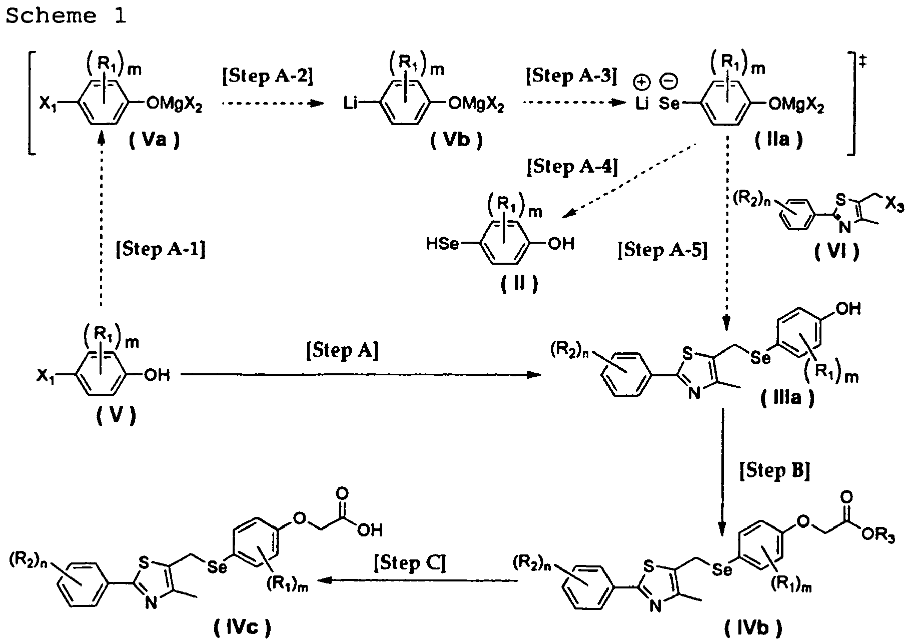

Alternatively, a benzyl may be introduced at the α-position of selenium. In this case, the hydroxyl group of the compound represented by the formula IIIa is protected and the compound is reacted with the compound represented by the formula VII (where X₄ is chlorine, bromine, iodine or other leaving group having good reactivity for nucleophilic substitution). Then, the protecting group is removed to obtain the compound represented by the formula IIIb, which is reacted with an alkyl halogen acetate, similarly as in the scheme 1, to obtain the compound represented by the formula IVd, which is ester hydrolyzed to obtain the compound represented by the formula IVe.

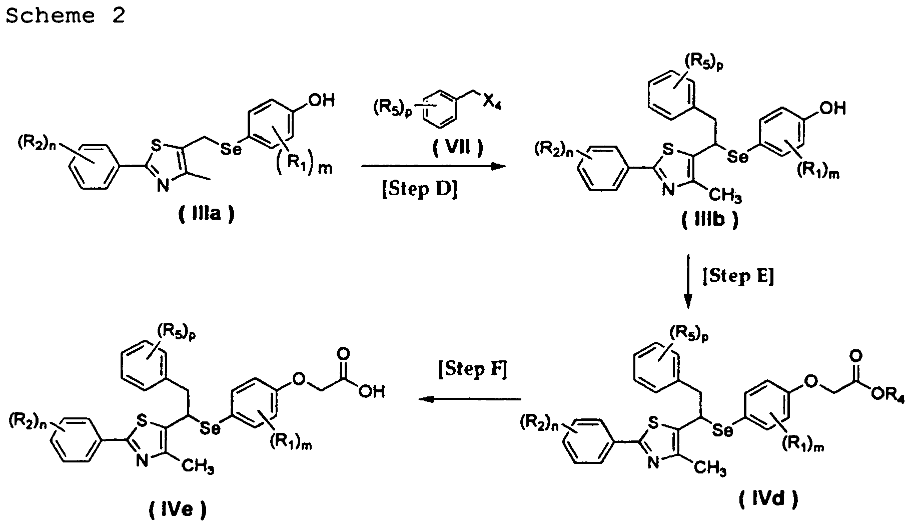

Hereunder is given a detailed description of the preparation method in accordance with the present invention.

### [Step A] Preparation of compound represented by formula IIIa

In order to obtain the compound represented by the formula IIIa, the phenol group of the compound represented by the formula V is protected using a Grignard reagent, without a special separation process, and the compound is reacted with an organometal reagent and selenium. In detail, this step comprises four substeps, which proceed at once.

The substeps are as follows.
(Substep A-1): At least one solvent selected from diethyl ether, tetrahydrofuran, hexane, heptane, etc. is used as anhydrous solvent. Among them, diethyl ether, tetrahydrofuran or a mixture solvent of diethyl ether and tetrahydrofuran is preferable.

For the Grignard reagent, methyl, ethyl, n-propyl, iso-propyl, n-butyl, sec-butylmagnesium chloride (R₂MgCl) or alkylmagnesium bromide (R₂MgBr) is used. Among them, iso-propylmagnesium chloride ((CH₃)₂CHMgCl) is the most preferable.

The reaction temperature may be different depending on the particular solvent used. In general, the reaction is performed in the temperature range from -20°C to 40°C, preferably from 0°C to room temperature (25°C). The reaction time may be different depending on the reaction temperature and the particular solvent used. In general, the reaction is performed for 10-60 minutes, preferably for 10-30 minutes.
(Substeps A-2 and A-3): For the organometal reagent for the halogen-metal substitution, n-butyllithium, sec-butyllithium, *tert*-butyllithium, etc. may be used. Among them, *tert*-butyllithium is preferable.

For the selenium, one in the form of fine powder is suitable. Selenium is directly added to the solvent.

The reaction temperature may be different depending on the particular solvent used. In general, the reaction is performed in the temperature range from -78 to 25°C. Preferably, the halogen-metal substitution is performed at -75 °C and the selenium introduction begins at -75°C and is performed while heating to room temperature (25°C). The halogen-metal substitution is performed for 10-30 minutes and the selenium introduction is performed for 30-90 minutes.
(Substep A-4): An acid is added to the compound represented by the formula IIa to identify the presence of -SeH. For the acid solution, a 1-3 N hydrochloric acid solution is preferable. The reaction is performed at 0-25 °C for 5-20 minutes.
(Substep A-5): The compound represented by the formula VI, 5-halogenmethyl-4-methyl-2-[4-(trifluoromethyl)phenyl]thiazole, is synthesized according to the known method (WO 03/106442). The halogen (X) of the compound represented by the formula VI may be chlorine, bromine or iodine. Among them, chlorine is preferable.

The reaction temperature may be different depending on the particular solvent used. In general, the reaction is performed in the temperature range from -78 to 25 °C, preferably from 0 to 10 °C. The reaction is performed for 10-120 minutes, preferably for 10-60 minutes.

### [Step B] Preparation of compound represented by formula IVb

In order to obtain the compound represented by the formula IVb, the compound represented by the formula IIIa is reacted with a haloacetate alkyl ester in the presence of a base.

The haloacetate alkyl ester is an easily available known compound and the halogen may be chlorine, bromine, iodine, etc. Bromoacetate methyl ester and/or bromoacetate ethyl ester are the most preferable haloacetate alkyl ester.

For the solvent, a water-soluble single solvent selected from *N*,*N*-dimethylformamide, *N*,*N*-dimethylacetamide, dimethylsulfoxide, acetonitrile, acetone, ethanol, methanol, etc. or a mixture solvent with 1-10 % of water is used. Among them, a mixture solvent of acetone or dimethylsulfoxide with 1-5 % of water is the most preferable.

For the base, either a weak base or a strong base may be used, as long as it does not negatively affect the reaction. For example, an alkyl metal hydride, such as sodium hydride and lithium hydride, an alkaline earth metal hydride such as potassium hydride, an alkali metal hydroxide strong base, such as sodium hydroxide and potassium hydroxide or an alkali metal carbonate such as lithium carbonate, potassium carbonate, potassium hydrocarbonate and cesium carbonate, may be used. An alkali metal carbonate is preferable and potassium carbonate is more preferable.

The reaction may be performed in any temperature range as long as the temperature is below the boiling point of the solvent. However, a relatively high temperature is not preferred because a side reaction may occur. In general, the reaction is performed at 0-60 °C. The reaction time may be different depending on the reaction temperature. In general, the reaction is performed for from 30 minutes to a day, preferably for 30-90 minutes.

### [Step C] Preparation of compound represented by formula IVc

The compound represented by the formula IVc is prepared from the compound represented by the formula IVb by carboxylic acid ester hydrolysis in a solution of a water-soluble inorganic salt and an alcohol.

For the solvent, an alcohol miscible with water, for example, methanol and ethanol, is used.

An alkali metal hydroxide base, such as lithium hydroxide, sodium hydroxide and potassium hydroxide, is used as prepared into a 0.1-3 N aqueous solution, depending on the particular alkali carboxylate. Acetic acid or a 0.1-3 N hydrochloric acid solution is used to obtain the carboxylate form of the compound represented by the formula IVc.

Preferably, the reaction is performed at a relatively low temperature in order to prevent any side reaction. In general, the reaction is performed in the temperature range from 0 °C to room temperature. The reaction time may be different depending on the reaction temperature. In general, the reaction is performed for from 10 minutes to 3 hours, preferably for from 30 minutes to 1 hour.

The resultant selenium containing compound represented by the formula IVc is an important material as ligand of PPARδ type proteins.

### [Step D] Preparation of compound represented by formula IIIb

The compound represented by the formula IIIb, in which the α-position of selenium is substituted with a benzyl group, may be prepared from the compound represented by the formula IIIa prepared in the step A by protecting its phenol group with TMSCl or TBDMSCl, dehydrogenating the active hydrogen at the α-position of selenium with a base like LDA, adding a benzyl halide derivative and removing the protecting group TMS or TBDMS.

The compounds represented by the formulas IVd and IVe are prepared by the steps E and F in the manner similar to the preparation of the compounds represented by the formulas IVb and IVc compound, respectively.

As described in detail above, the organoselenium compounds of the present invention have ligands having an activity for PPARδ and thus they are highly probable candidates for treatments for cardiovascular diseases, cholesterol depressants, diabetic treatments and treatments for obesity. The preparation method in accordance with the present invention is useful for the preparation of the organoselenium compounds.

### EXAMPLES

Hereinafter, the present invention is described in detail with reference to the preferred examples. However, the following examples are only for the understanding of the present invention and the present invention is not limited to or by them.

### Example 1: Preparation of 4-hydroseleno-2-methylphenol (II) (reference example)

400 mg (1.7 mmol) of 4-iodo-2-methylphenol was dissolved in 30 mL of anhydrous tetrahydrofuran under nitrogen atmosphere and the temperature was maintained at 0 °C. 935 µL of isopropylmagnesium chloride (2 M ether solution, 1.1 equivalent) was slowly added and reaction was performed for 10 minutes. The reaction solution was cooled to -78 °C and 2.2 mL of *tert*-butyllithium (1.7 M heptane solution, 2.2 equivalents) was slowly added dropwise and reaction was performed for 20 minutes. 134 mg of selenium (1.7 mmol, 1.0 equivalent) was slowly added and reaction was performed until the temperature of the reaction mass reached room temperature. 30 mL of an ammonium chloride solution and 1 N hydrochloric acid were added. The organic layer was separated and moisture was removed with magnesium sulfate. After filtration, the solvent was removed by distillation under reduced pressure. The residue was purified with hexane/ethyl acetate (v/v = 2/1) by silica gel column chromatography to obtain 302 mg of the target compound (yield: 95 %).
¹H NMR (300 MHz, CDCl₃) δ 7.35(d, 1H, *J* = 1.3 Hz), 7.29(dd, 1H, *J* = 8.2, 2.2 Hz), 6.67(d, 1H, *J* = 8.2 Hz), 4.84(br s, 1H), 2.21(s, 3H).
¹³C NMR (75.5 MHz, CDCl₃) δ 154.8, 137.3, 133.6, 125.2, 122.4, 115.9, 16.0.

### Example 2: Preparation of 4-[[2-[4-(trifluoromethyl)phenyl]-4-methylthiazol-5-yl]methylselanyl]-2-methylphenol (IIIa)

1.17 g (5.0 mmol) of 4-iodo-2-methylphenol was dissolved in 80 mL of anhydrous tetrahydrofuran under nitrogen atmosphere and the temperature was maintained at 0 °C. 2.75 mL of isopropylmagnesium chloride (2 M ether solution, 1.1 equivalent) was slowly added and reaction was performed for 10 minutes. The reaction solution was cooled to -78 °C and 6.47 mL of tert-butyllithium (1.7 M heptane solution, 2.2 equivalents) was slowly added dropwise and reaction was performed for 20 minutes. 395 mg of selenium (5.0 mmol, 1.0 equivalent) was slowly added and reaction was performed until the temperature of the reaction mass reached 15 °C. 40 minutes later, 1.46 g of the compound represented by the formula VI, 5-chloromethyl-4-methyl-2-[(4-trifluoromethyl)phenyl]-thiazole, (5.0 mmol, 1.0 equivalent) dissolved in 5 mL of anhydrous THF was slowly added at the same temperature. After about 30 minutes of reaction, 100 mL of an ammonium chloride solution was added to terminate the reaction. The organic layer was separated and moisture was removed with magnesium sulfate. After filtration, the solvent was removed by distillation under reduced pressure. The residue was purified with hexane/ethyl acetate (v/v = 3/1) by silica gel column chromatography to obtain 2.05 g of the target compound (yield: 93 %).
¹H NMR (300 MHz, CDCl₃) δ 7.95(d, 2H, *J* = 8.1 Hz), 7.64(d, 2H, *J* = 8.2 Hz), 7.28(d, 1H, *J* = 1.4 Hz), 7.09(dd, 1H, *J* = 8.2, 1.9 Hz), 6.59(d, 1H, *J* = 8.2 Hz), 4.09(s, 2H), 2.19(s, 3H), 2.05(s, 3H).
¹³C NMR (75.5 MHz, CDCl₃) δ 163.7, 155.4, 151.5, 139.3, 135.5, 135.3(q, *J* = 33 Hz) 132.5, 126.8, 126.3(m), 125.7, 118.5, 115.9, 23.3, 16.1, 14.9.

### Example 3: Preparation of ethyl 2-[4-[[2-[4-(trifluoromethyl)phenyl]-4-methylthiazol-5-yl]methylselanyl]-2-methylphenoxy]acetate (IVb)

1.0 g of 4-[[2-[4-(trifluoromethyl)phenyl]-4-methylthiazol-5-yl]methylselanyl]-2-methylphenol (2.26 mmol) prepared in Example 2 was mixed well with 50 mL of acetone containing 5 % of water and 719 mg of potassium carbonate (5.2 mmol, 2.3 equivalents) at room temperature. 376 µL of bromoacetic acid ethyl ester (3.4 mmol, 1.5 equivalent) was added and the mixture was strongly stirred for 4 hours. After the reaction was completed, extraction was performed using brine and ethyl acetate and moisture was removed with magnesium sulfate. After filtration, the solvent was removed by distillation under reduced pressure and the residue was purified with hexane/ethyl acetate (v/v = 5:1) by silica gel column chromatography to obtain 1.19 g of the target compound (yield: 99 %).
¹H NMR (300 MHz, CDCl₃) δ 7.96(d, 2H, *J* = 8.1 Hz), 7.65(d, 2H, *J* = 8.3 Hz), 7.29(d, 1H, *J* = 1.4 Hz), 7.23(dd, 1H, *J* = 8.4, 2.1 Hz), 6.56(d, 1H, *J* = 8.4 Hz), 4.62(s, 2H), 4.25(q, 2H, *J* = 14.3, 7.1 Hz), 4.12(s, 2H), 2.23(s, 3H), 2.14(s, 3H), 1.28(t, 3H, *J* = 7.1 Hz).
¹³C NMR (75.5 MHz, CDCl₃) δ 169.1, 162.9, 157.1, 151.6, 138.8, 137.3, 134.8, 131.9, 131.6(q, *J* = 33 Hz), 128.9, 126.2(m), 120.0, 112.1, 65.9, 61.8, 23.3, 16.4, 15.2, 14.6.

### Example 4: Preparation of 2-[4-[[2-[4-(trifluoromethyl)phenyl]-4-methylthiazol-5-yl]methylselanyl]-2-methylphenoxy]acetic acid (IVb, HK101225)

500 mg of ethyl 2-[4-[[2-[4-(trifluoromethyl)phenyl]-4-methylthiazol-5-yl]methylselanyl]-2-methylphenoxy]acetate (1.0 mmol) prepared in Example 3 was mixed well with 50 mL of ethanol and 3.5 mL of a 3 N sodium hydroxide solution was added. Stirring was performed for 30 minutes at room temperature. When the reaction was completed, pH was adjusted to 2.0 with 2 N HCl. About 80 % of ethanol was removed by distillation under reduced pressure. After extraction using brine and ethyl acetate and filtration, the solvent was removed by distillation under reduced pressure and the residue was purified by LH-20 column chromatography to obtain 495 mg of the target compound (yield: 99 %).
¹H NMR (300 MHz, CDCl₃) δ 9. 92 (br s, 1H), 7.93(d, 2H, *J* = 8.2 Hz), 7.65(d, 2H, *J* = 8.4 Hz), 7.29(d, 1H, *J* = 1.4 Hz), 7.19(dd, 1H, *J* = 8.4, 2.1 Hz), 6.58(d, 1H, *J* = 8.4 Hz), 4.66(s, 2H), 4.10(s, 2H), 2.21(s, 3H), 2.07(s, 3H).
¹³C NMR (150.9 MHz, CDCl₃) δ 173.2, 163.5, 156.8, 151.5, 138.9, 136.8, 134.9, 132.3, 131.8(q, *J* = 33 Hz), 128.9, 126.9, 126.3(m), 120.1, 112.0, 65.4, 23.1, 16.4, 14.8.

### Example 5: Preparation of 4-[1-[4-methyl-2-(4-trifluoromethylphenyl)-thiazol-5-yl]-2-phenylethylselanyl]-2-methyl-phenol (IIIb)

### A: Preparation of 5-[4-(tert-butyldimethylsilanyloxy)-3-methyl-phenylselanylmethyl]-4-methyl-2-[(4-trifluoromethyl)phenyl]-thiazole

200 mg of 4-[[2-[4-(trifluoromethyl)phenyl]-4-methylthiazol-5-yl]methylselanyl]-2-methylphenol (0.45 mmol) prepared in Example 2 and 77 mg of imidazole (1.13 mmol, 2.5 equivalents) were completely dissolved in anhydrous dimethylformamide (5 mL). 102 mg of *tert-*butylmethylsilyl chloride (0.67 mmol, 1.5 equivalent) was slowly added and the mixture was stirred for 4 hours at room temperature. After the reaction was completed, the organic layer was extracted with water (20 mL) and ethyl ether (15 mL) and was washed with water (20 mL). The organic layer was dried with magnesium sulfate and 238 mg of 5-[4-(*tert*-butyldimethylsilanyloxy)-3-methyl-phenylselanylmethyl]-4-methyl-2-[(4-trifluoromethyl)phenyl]-thiazole, with the phenol group protected, was obtained by silica gel chromatography (yield: 95%).

### B: Preparation of 5-[1-[4-(tert-butyldimethylsilyloxy)-3-methyl-phenylselanyl]-2-phenylethyl]-4-methyl-2-[4-(trifluoromethyl)phenyl]-thiazole

150 mg of 5-[4-(*tert*-butyldimethylsilanyloxy)-3-methyl-phenylselanylmethyl]-4-methyl-2-[(4-trifluoromethyl)phenyl]-thiazole (0.27 mmol) prepared in A was dissolved in anhydrous tetrahydrofuran (5 mL) under nitrogen atmosphere. The reaction solution was cooled to - 78°C and 240 µL of lithium diisopropylamide (lithium diisopropylamide, 2 M heptane/tetrahydrofuran/ethylbenzene solution, 2.0 equivalents) was slowly added. Reaction was performed for 30 minutes at the same temperature while adding 32 µL of benzyl bromide (0.27 mmol, 1.0 equivalent), maintaining the color of the reaction solution deep blue. A saturated ammonium chloride solution (10 mL) was to the reaction solution. After extraction with ethyl acetate (10 mL), the organic layer was washed with brine. The organic layer was dried with magnesium sulfate and the concentrated residue was purified by silica gel chromatography to obtain 141 mg of the target compound (yield: 81 %).
¹H NMR (600 MHz, CDCl₃) δ 7.99(d, 2H, J = 8.3 Hz), 7.12-7.26(m, 7H), 6.64(d, 2H, J = 8.2 Hz), 4.73(dd, 1H, J = 9.8, 5.6 Hz), 3.44(dd, 1H, J = 14.0, 5.6 Hz), 3.25(dd, 1H, J = 14.0, 9.8 Hz), 2.12(s, 3H), 1.91(s, 3H), 1.03(s, 9H), 0.21(s, 6H).

### C: Preparation of 9-[1-[4-methyl-2-(4-trifluoromethylphenyl)-thiazol-5-yl]-2-phenylethylselanyl]-2-methylphenol

100 mg of 5-[1-[4-(*tert*-butyldimethylsilyloxy)-3-methyl-phenylselanyl]-2-phenylethyl]-4-methyl-2-[4-(trifluoromethyl)phenyl]-thiazole (0.15 mmol) prepared in B was completely dissolved in tetrahydrofuran (10 mL) at room temperature. 180 µL of tetrabutylammonium fluoride (TBAF) (0.18 mmol, 1 M tetrahydrofuran solution, 1.2 equivalent) was slowly added at the same temperature. After 1 hour of reaction, the organic layer was extracted with a saturated ammonium chloride solution (10 mL) and ethyl acetate (10 mL) and dried with magnesium sulfate. After filtration, the solvent was removed by distillation under reduced pressure and the concentrated residue was purified by silica gel chromatography to obtain 79 mg of the target compound (yield: 99%).
¹H NMR (300 MHz, CDCl₃) δ 7.94(d, 2H, J = 8.2 Hz), 7.63(d, 2H, J = 8.3), 7.00-7.26(m, 7H), 6.50(d, 1H, J = 8.1 Hz), 6.32(br, 1H), 4.67(dd, 1H, J = 9.8, 5.7 Hz), 3.42(dd, 1H, J = 13.9, 5.7 Hz), 3.22(dd, 1H, J = 13.9, 9.8 Hz), 2.13(s, 3H), 1.78(s, 3H).

### Example 6: Preparation of ethyl [4-[1-[4-methyl-2-(4-trifluoromethylphenyl)-thiazol-5-yl]-2-phenylethylselanyl]-2-methyl-phenoxy]-acetate (IVd)

100 mg of 4-[1-[4-methyl-2-(4-trifluoromethylphenyl)-thiazol-5-yl]-2-phenylethylselanyl]-2-methylphenol (0.19 mmol) prepared in Example 5 was completely dissolved in acetone (10 mL) containing 5 % of water and 66 mg of potassium carbonate (0.475 mmol, 2.5 equivalents) was added at room temperature. 28 µL of bromoacetic acid ethyl ester (0.25 mmol. 3 equivalents) was added at the same temperature and the mixture was stirred for 4 hours. After the reaction was completed, the organic layer was extracted with brine (10 mL) and ethyl acetate (10 mL). Then, the organic layer was dried with magnesium sulfate. The solvent was removed by distillation under reduced pressure and the residue was purified by silica gel chromatography to obtain 103 mg (yield: 88 %) of the target compound.
¹H NMR (300 MHz, CDCl₃) δ 7.97(d, 2H, *J* = 8.2 Hz), 7.65(d, 2H, *J* = 8.4 Hz), 7.07-7.25(m, 7H), 6.53(d, 2H, *J* = 8.1 Hz)), 4.69(dd, 1H, *J* = 9.8, 6.0 Hz), 4.59(s, 2H), 4.23(q, 1H, *J* = 14.2, 7.1Hz), 3.40(dd, 1H, *J* = 13.8, 6.0 Hz), 3.20(dd, 1H, *J* = 13.8, 9.8 Hz), 2.26(s, 3H), 1.85(s, 3H), 1.28(t, 3H, *J* = 7.0 Hz).

### Example 7: Preparation of [4-[1-[4-methyl-2-(4-trifluoromethylphenyl)-thiazol-5-yl]-2-phenylethylselanyl]-2-methylphenoxy]-acetic acid (IVe)

100 mg of [4-[1-[4-methyl-2-(4-trifluoromethylphenyl)-thiazol-5-yl]-2-phenylethylselanyl]-2-methylphenoxy]-acetate (0.16 mmol) prepared in Example 6 was completely dissolved in ethanol (10 mL) and 200 µL of a 1 N sodium hydroxide solution was added. After stirring for 30 minutes at room temperature, when the reaction was completed, pH was adjusted to 3.0 with a 2 N HCl solution. The reaction solvent was removed under reduced pressure and the organic layer was extracted with brine and ethyl acetate. The residue was purified by LH-20 resin column chromatography to obtain 71 mg of the target compound (yield: 75 %).
¹H NMR (300 MHz, CDCl₃) δ 7.97(d, 2H, *J* = 8.2 Hz), 7.65(d, 2H, *J* = 8.4 Hz), 7.07-7.25(m, 7H), 6.53(d, 2H, *J* = 8.1 Hz)), 4.69(dd, 1H, *J* = 9.8, 6.0 Hz), 4.59(s, 2H), 3.40(dd, 1H, *J* = 13.8, 6.0 Hz), 3.20(dd, 1H, *J* = 13.8, 9.8 Hz), 2.26(s, 3H), 1.85(s, 3H).

### Examples 7 to 22

The organoselenium compounds presented in Table 1 and Table 2 below were prepared in the similar manner as in Examples 1 to 6.

**Table 1**

| | |
|---|---|
| 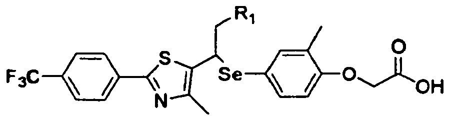 | |

| R₁ | 1H NMR (300 MHz, CDCl3) |
|---|---|
| 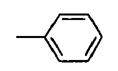 | 7.97(d, 2H, *J* = 8.2 Hz), 7.65(d, 2H, *J* = 8.4 Hz), 7.07-7.25(m, 7H), 6.53(d, 2H, *J* = 8.1 Hz)), 4.69(dd, 1H, *J* = 9.8, 6.0 Hz), 4.59(s, 2H), 3.40(dd, 1H, *J* = 13.8, 6.0 Hz), 3.20(dd, 1H, *J* = 13.8, 9.8 Hz), 2.26(s, 3H), 1.85(s, 3H) |
| 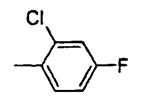 | 7.97(d, 2H, *J* = 8.2 Hz), 7.65(d, 2H, *J* = 8.5 Hz), 7.11-7.27(m, 4H), 6.90(m, 1H), 6.53(d, 1H, *J* = 8.1 Hz), 4.92(t, 1H, *J =* 7.8 Hz), 4.59(s, 2H), 3.45(d, 2H, *J* = 7.9 Hz), 2.19(s, 3H), 1.91(s, 3H) |
| 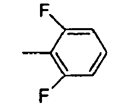 | 7.97(d, 2H, *J* = 8.2 Hz), 7.65(d, 2H, *J* = 8.3 Hz), 7.11-7.26(m, 3H), 6.79(m, 2H), 6.53(d, 1H, *J* = 8.0 Hz), 4.85(t, 1H, *J* = 8.2 Hz), 4.62(s, 2H), 3.45(d, 2H, *J* = 8.2 Hz), 2.19(s, 3H), 1.97(s, 3H) |
| 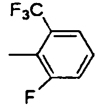 | 7.9(d, 2H, *J* = 8.2 Hz), 7.65(d, 2H, *J* = 8.3 Hz), 7.14-7.46(m, 5H), 6.53(d, 1H, *J* = 8.0 Hz), 4.84(t, 1H, *J* = 7.8 Hz), 4.62(s, 2H), 3.50(d, 2H, *J* = 7.8 Hz), 2.18(s, 3H), 1.83(s, 3H) |
| 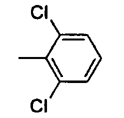 | 7.98(d, 2H, *J* = 8.2 Hz), 7.65(d, 2H, *J* = 8.5 Hz), 7.07-7.27(m, 5H), 6.52(d, 1H, *J* = 8.1 Hz), 4.98(t, 1H, *J* = 7.8 Hz), 4.58(s, 2H), 3.50(m, 2H), 2.18(s, 3H), 1.84(s, 3H) |
| 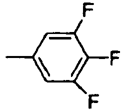 | 7.97(d, 2H, *J* = 8.2 Hz), 7.67(d, 2H, *J* = 8.1 Hz), 7.18-7.26(m, 2H), 6.69-6.76(m, 2H), 6.54(d, 1H, *J* = 8.1 Hz), 4.60(m, 3H), 3.31(dd, 1H, *J* = 14.2, 5.9 Hz), 3.15(dd, 1H, *J* = 14.2, 9.7 Hz), 2.19(s, 3H), 1.95(s, 3H) |
| 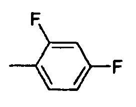 | 7.97(d, 2H, *J* = 8.1 Hz), 7.65(d, 2H, *J* = 8.2 Hz), 7.18-7.26(m, 2H), 7.03(m, 1H), 6.72(m, 1H), 6.53(d, 1H, *J* = 8.1 Hz), 4.74(dd, 1H, *J* = 9.5, 6.2 Hz) 4.59(s, 2H), 3.43(dd, 1H, *J* = 14.1, 6.2 Hz), 3.18(dd, 1H, *J* = 14.1, 9.5 Hz), 2.19(s, 3H), 1.93(s, 3H) |
| 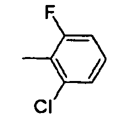 | 7.98(d, 2H, *J* = 8.2 Hz), 7.65(d, 2H, *J* = 8.3 Hz), 7.08-7.26(m, 3H), 6.90(m, 1H), 6.53(d, 1H, *J* = 8.1 Hz), 4.93(t, 1H, *J* = 7.9 Hz), 4.59(s, 2H), 3.44(d, 1H, *J* = 7.9 Hz), 2.19(s, 3H), 1.91(s, 3H) |

**Table 2**

| | |
|---|---|
| 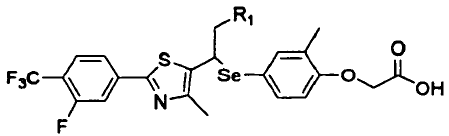 | |

| R₁ | 1H NMR (δ) |
|---|---|
| 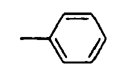 | 7.62-7.74(m, 3H), 7.07-7.25(m, 7H), 6.53(d, 1H, *J* = 8.1 Hz), 4.69(dd, 1H, *J* = 9.8, 5.7 Hz), 4.62(s, 2H), 3.40(dd, 1H, *J* = 13.9 5.7 Hz), 3.23(dd, 1H, *J* = 13.9, 9.8 Hz), 2.24(s, 3H), 1.85(s, 3H) |
| 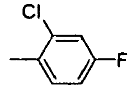 | 7.60-7.76(m, 3H), 7.08-7.27(m, 4H), 6.89(m, 1H), 6.53(d, 1H, *J* = 8.1 Hz), 4.91(t, 1H, *J* = 7.9 Hz), 4.62(s, 2H), 3.46(d, 1H, *J* = 7.9 Hz) 2.22(s 3H), 1.91(s, 3H) |
| 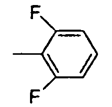 | 7.59-7.75(m, 3H), 7.10-7.29(m, 4H), 6.80(m, 1H), 6.53(d, 1H, *J* = 8.1 Hz), 4.91(t, 1H, *J* = 8.4 Hz), 4.59(s, 2H), 3.35(m, 2H), 2.19(s, 3H), 1.96(s, 3H) |
| 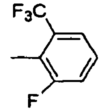 | 7.60-7.77(m, 3H), 7.15-7.47(m, 5H), 6.52(d, 1H, *J* = 8.1 Hz), 4.82(t, 1H, *J* = 7.9 Hz), 4.59(s, 2H), 3.48(m, 2H), 2.18(s, 3H), 1.83(s, 3H) |
| 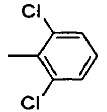 | 7.60-7.76(m, 3H), 7.07-7.26(m, 5H), 6.52(d, 1H, *J* = 8.2 Hz), 4.82(t, 1H, *J* = 7.4 Hz), 4.59(s, 2H), 3.59(m, 2H), 2.19(s, 3H), 1.83(s, 3H) |
| 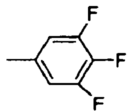 | 7.61-7.74(m, 3H), 7.17-7.26(m, 2H), 6.71(m, 2H), 6.54(d, 1H, *J* = 8.1 Hz), 4.59(m, 3H), 3.32(dd, 1H, *J* = 14.2, 5.9 Hz), 3.16(dd, 1H, *J* = 14.2, 9.6 Hz). 2.19(s, 3H), 1.96(s, 3H) |
| 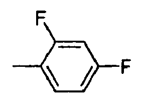 | 7.60-7.75(m, 3H), 7.18-7.21(m, 2H), 6.98-7.07(m, 1H), 6.69-6.78(m, 2H), 6.53(d, 1H, *J* = 8.1 Hz), 4.74(dd, 1H, *J* = 9.5, 6.2 Hz), 4.60(s, 2H), 3.42(dd, 1H, *J* = 14.1, 6.3 Hz), 3.23(dd, 1H, *J* = 14.1, 9.5 Hz), 2.23(s, 3H), 1.94(s, 3H) |
| 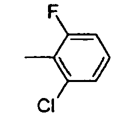 | 7.59-7.76(m, 3H), 7.11-7.27(m, 4H), 6.89(m, 1H), 6.53(d, 1H, *J* = 8.1 Hz), 4.91(t, 1H, *J* = 7.9 Hz), 4.59(s, 2H), 3.45(d, 1H, *J* = 7.9 Hz), 2.19(s, 3H), 1.91(s, 3H) |

### Testing Example 1: Activity and toxicity test

Activity of HK101225 for PPARδ was tested by transfection assay. In addition, selectivity for PPARα and PPARγ subtypes tested. Toxicity test was performed by MTT assay and *in vivo* activity was confirmed by animal test.

### Transfection assay

Transfection assay was performed using CV-1 cells. The cells were cultured with a DMEM medium containing 10 % FBS, DBS (delipidated) and 1 % penicillin/streptomycin in 37 °C of a transfection assay culture system containing 5 % of carbon dioxide on a 96-well plate. Test was performed in four stages - cell inoculation, transfection, treatment with the compound of the present invention and confirmation. The CV-1 cells were inoculated on the 96-well plate, 5,000 cells/well. Transfection was performed 24 hours later. Full-length PPARs plasmid DNA, reporter DNA, which has luciferase activity and thus enables confirmation of the activity for PPARs, and β-galactosidase DNA, which offers effective information on the transfection, were used. HK101225 prepared in Example 4 was dissolved in dimethylsulfoxide (DMSO) and treated to the cells at various concentrations. After 24 hours of culturing in an incubator, the cells were lysed with a lysis buffer and luciferase activity and β-galactosidase activity were measured using a luminometer and a microplate reader. The measured luciferase activity was calibrated with the β-galactosidase activity. The result was depicted on a graph and EC₅₀ was determined.

**Table 3**

| Compound | EC₅₀ PPARα | EC₅₀ PPARγ | EC₅₀ PPARδ |
|---|---|---|---|
| HK101225 (Example 4) | > 10000 nM | > 10000 nM | 1.87 nM |

As seen in Table 3, the organoselenium compound of the present invention was highly selective for EC₅₀ PPARδ.

The compounds presented in Tables 1 and 2 and ethyl esters thereof showed at least 10,000 times of selectivity for PPARα and PPARγ and EC₅₀ for PPARδ was in the range from 500 pM to 10 nM.

### MTT assay

Toxicity was tested by MTT assay for HK101225 prepared in Example 4. MTT is a water-soluble, yellow substance. However, when introduced in a living cell, it is transformed into a water-insoluble, purple crystal by the mitochondrial dehydrogenase. Cell toxicity can be tested by dissolving this substance in dimethylsulfoxide and measuring light absorbance at 550 nm. The test was performed as follows.

CV-1 cells were inoculated on a 96-well plate, 5,000 cells/well. After culturing for 24 hours in a humidified culture system of 37 °C containing 5 % of carbon dioxide, the cells were treated with HK101225 at various concentrations. MTT reagent was added after 24 hours of culturing. After culturing for about 15 minutes, the purple crystal was dissolved in dimethylsulfoxide and absorbance was measured with a microplate reader to confirm cell toxicity.

HK101225 showed no toxicity even at the concentration of 50,000 times of the EC₅₀ (1.87 nM) value. Other compounds presented in Tables 1 and 2 and ethyl esters thereof showed no toxicity at the concentration of 10,000 times of the EC₅₀ value.

### Animal test

### Prevention of obesity

Animal test was performed with mice in order to confirm the in vivo effect of HK101225. 14-weeks old C57BL/6 mice (SLC Co.) were used and a feed containing 35 % of fat was given to induce obesity. A vehicle, GW501516 (10 mg/kg/day) and HK101225 (10 mg/kg/day) were orally administered over a 78-day period of high-fat diet. The mice administered with the vehicle showed a 102 % increase in body weight, but the body weight of the mice administered with GW501516 and HK101225 increased by only 21 %, about 1/5 of the increase in body weight of the group to which the vehicle was administered. Thus, it was confirmed that HK101225 has a strong prevention effect against obesity.

### Improvement of diabetes

GTT (glucose tolerance test) was performed in order to confirm the diabetic improvement effect of the compound of the present invention. A vehicle, GW501516 and HK101225 were orally administered to mice over a 78-day period. Then, glucose (1.5 g/kg) was abdominally administered and change of blood sugar level with time was checked. The group to which HK101225 was administered showed lower fasting blood sugar level than the groups to which the vehicle and GW501516 were administered. The group to which HK101225 was administered showed a rapid decrease of blood sugar level between 20 and 40 minutes and showed a complete glucose clearance after 100 minutes. In contrast, the group to which the vehicle was administered did not maintain a normal blood sugar level even after 120 minutes. The group to which GW501516 was administered showed a blood sugar level lower than that of the vehicle group, but did not restore to the normal level. Thus, it was confirmed that HK101225 is more effective in improving diabetes than GW501516.

The selenium containing compound represented by the formula IV or a pharmaceutically available salt of the compound is useful as activator composition for peroxisome proliferator activated receptor δ (PPARδ). Also, the selenium containing compound represented by the formula IV or a pharmaceutically available salt of the compound is useful as medical composition of cardiovascular diseases, cholesterol depressant, diabetic treatment or treatment for obesity, health food supplement, health drink, food ingredient and functional cosmetic for treatment.

The pharmaceutically available salt of the compound represented by the formula IV may be a carboxylate or any other pharmaceutically available salt, the alkali metal ion being an alkaline earth metal, such as Li⁺, Na⁺, K⁺ and Ca⁺.

The therapeutic dosage of the compound represented by the formula IV or the pharmaceutically available salt thereof may vary depending on the particular compound, administration method, particular patient and particular disease to be treated. The compound may be administered orally or locally depending on the preparation type. In case of oral administration, the compound may be prepared in any form, including tablet, powder, dry syrup, chewable tablet, granule, chewing gum, capsule, soft capsule, pill, drink and sublingual tablet. The tablet comprising the compound of the present invention may be administered to a patient by any type or method that is biologically acceptable, that is via an oral route. Alternatively, it may be administered by other type or method, depending on the characteristics of the disease to be treated or prevented, the progress of the disease or other circumstances. In case the composition comprising the compound of the present invention is a tablet, it may comprise at least one pharmaceutically acceptable excipient. The proportion and property of the excipient may be determined considering the solubility or other chemical properties of the tablet, route of administration and other pharmaceutical standards.

The present invention provides a novel organoselenium, which is useful as ligand having activity for PPARδ activity, and a preparation method thereof.

The present invention provides a convenient way of preparing the organoselenium compound by protecting the phenol group of a phenol compound with a Grignard reagent, without introduction of a special protecting group, reacting it with an organometal reagent without separation and subsequently reacting it with selenium (Se).

While the present invention has been described in detail with reference to the preferred embodiments, those skilled in the art will appreciate that various modifications and substitutions can be made thereto without departing from the scope of the present invention as set forth in the appended claims.

## Claims

1. An organoselenium compound represented by the formula I below: 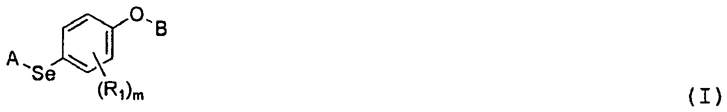 where
A is 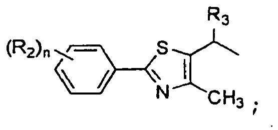 B is hydrogen or 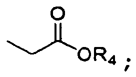 R₁ is independently C₁-C₄ alkyl, C₁-C₄ alkyloxy, C₁-C₄ alkylthioxy, C₁-C₄ alkylamine, fluorine or chlorine;
R₂ is independently C₁-C₄ alkyl, C₁-C₄ alkyl substituted with at least one halogen or halogen;
R₃ is hydrogen, C₁-C₄ alkyl or 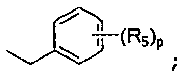 R₄ is hydrogen, alkali metal ion or C₁-C₇ alkyl or aryl;
R₅ is independently C₁-C₄ alkyl, C₁-C₄ alkyl substituted with at least one halogen or halogen;
m is an integer from 0 to 4;
n is an integer from 0 to 5; and
p is an integer from 0 to 5.

2. The organoselenium compound of Claim 1, which is a racemic compound or an optical isomer represented by the formula III below: 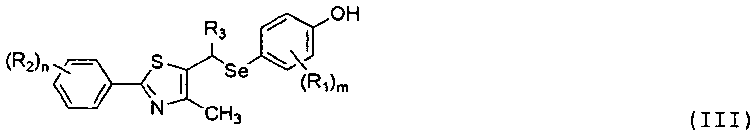 where R₁ to R₃, R₅, m, n and p are the same as defined in the formula 1.

3. The organoselenium compound of Claim 1, which is a racemic compound or an optical isomer represented by the formula IV below: 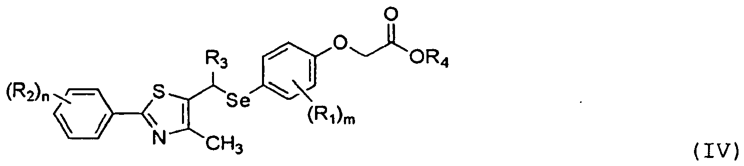 where R₁ to R₅, m, n and p are the same as defined in the formula 1.

4. The organoselenium compound of Claim 1, which is a racemic compound or an optical isomer represented by the formula IVa below: 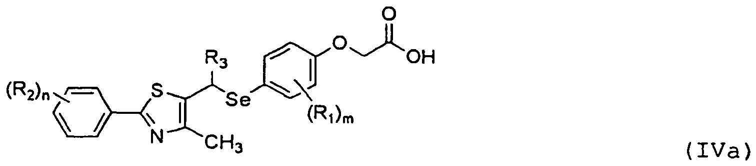 where R₁ to R₃, R₅, m, n and p are the same as defined in the formula 1.

5. The organoselenium compound of Claim 5, wherein:
R₁ is independently methyl or ethyl;
R₂ is independently methyl, ethyl, CF₃, F or Cl;
R₃ is hydrogen or 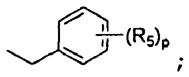
R₅ is independently CF₃, F or Cl;
m is an integer from 0 to 2;
n is an integer from 0 to 3; and
p is an integer from 0 to 3.

6. A preparation method of preparing the organoselenium compound of claim 1 comprising the steps of:
a) reacting the compound represented by the formula V below with a Grignard reagent and reacting it with an organometal reagent to obtain the organometal compound represented by the formula Vb below;
b) subsequently reacting the organometal compound represented by the formula Vb with selenium(Se) to obtain the metal-selenium compound represented by the formula IIa below; and
c) subsequently reacting the selenium compound represented by the formula IIa with the thiazole compound represented by the formula VI below to obtain the compound represented by the formula IIIa below: 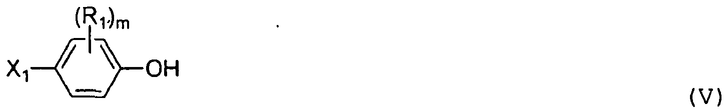 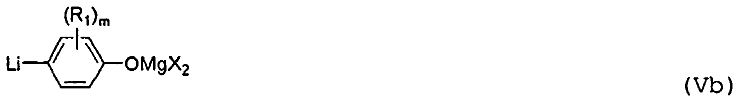 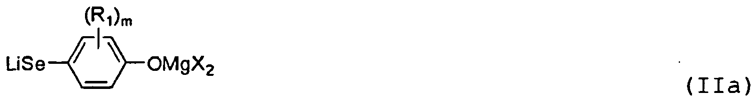 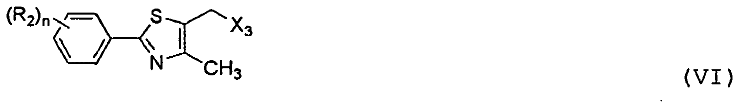 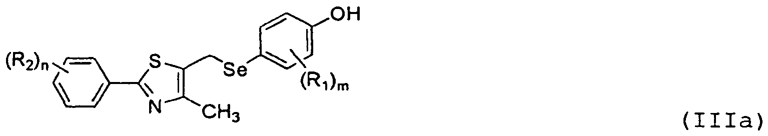 where X₁ is bromine or iodine, X₂ is chlorine, bromine or iodine, X₃ is chlorine, bromine, iodine or other leaving group having good reactivity for nucleophilic substitution and other substituents are the same as defined in the formula 1.

7. The preparation method of Claim 6, which further comprises the steps of
e) reacting the compound represented by the formula IIIa below with an alkyl halogen acetate to obtain the ester compound represented by the formula IVb below; and
f) hydrolyzing the ester compound represented by the formula IVb to obtain the compound represented by the formula IVc below: 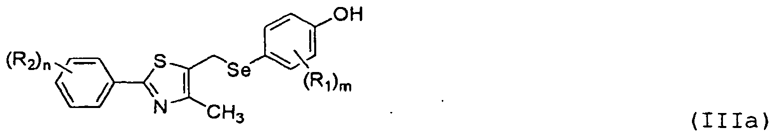 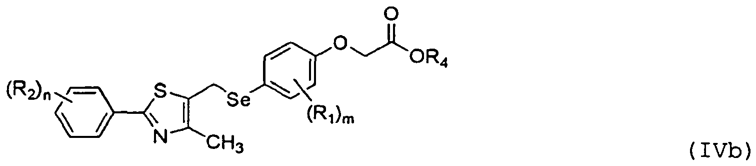 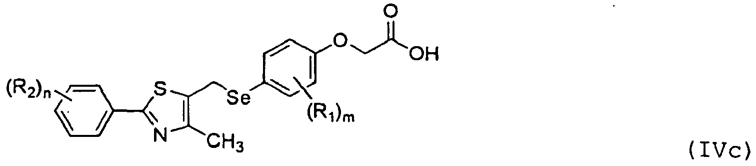 where R₁ to R₃, m, n and p are the same as defined in the formula 1 and R₄ is C₁-C₇ alkyl or aryl.

8. The preparation method of Claim 6, which further comprises the steps of:
g) protecting the hydroxyl group of the compound represented by the formula IIIa below, reacting the compound with the compound represented by the formula VII below in a basic condition and removing the hydroxyl protecting group to obtain the compound represented by the formula IIIb below;
h) reacting the compound represented by the formula IIIb with an alkyl halogen acetate to obtain the ester compound represented by the formula IVd below; and
i) hydrolyzing the ester compound represented by the formula IVd to obtain the compound represented by the formula IVe below: 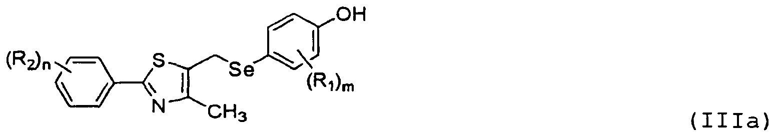 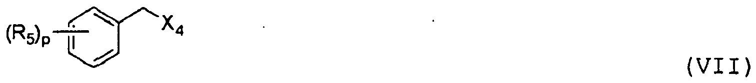 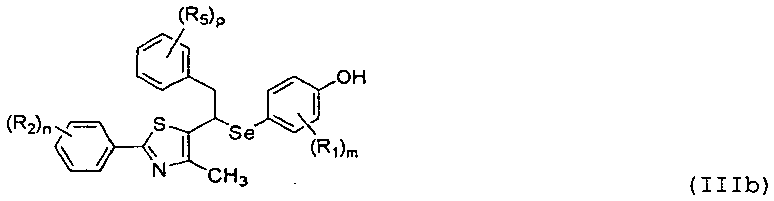 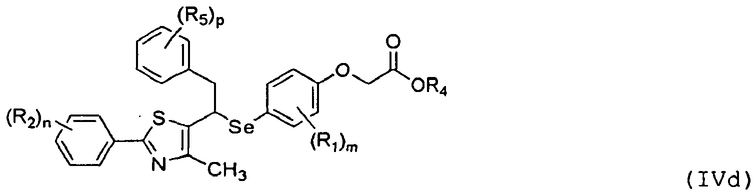 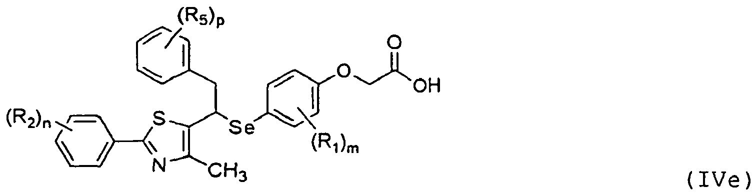 where R₁ to R₃, R₅, m, n and p are the same as defined in the formula 1, R₄ is C₁-C₇ alkyl or aryl and X₄ is chlorine, bromine, iodine or other leaving group having good reactivity for nucleophilic substitution.

9. A medical composition for use in the treatment of cardiovascular diseases,
diabetes or obesity, or for use as cholesterol depressant comprising the selenium containing compound represented by the formula IV of claim 3 or a pharmaceutically available salt of the compound as active ingredient.

10. A health food supplement, health drink, food ingredient or functional cosmetic comprising the selenium containing compound represented by the formula IV of claim 3 or a pharmaceutically available salt of the compound as active ingredient.

11. A peroxisome proliferator activated receptor δ (PPARδ) activator composition comprising the selenium containing compound represented by the formula IV of claim 3 or a pharmaceutically available salt of the compound as active ingredient.

## Patentansprüche

1. Organoselenverbindung, die durch die untenstehende Formel I dargestellt wird: 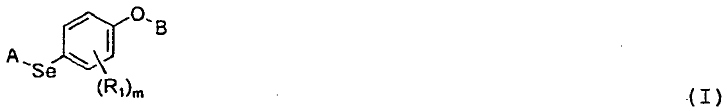 worin A 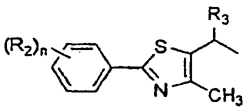 ist;
B Wasserstoff oder 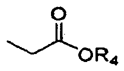 ist;
R₁ unabhängig C₁-C₄-Alkyl, C₁-C₄-Alkyloxy, C₁-C₄-Alkylthioxy, C₁-C₄-Alkylamin, Fluor oder Chlor ist;
R₂ unabhängig C₁-C₄-Alkyl, C₁-C₄-Alkyl, das mit wenigstens einem Halogen substituiert ist, oder Halogen ist;
R₃ Wasserstoff, C₁-C₄-Alkyl oder 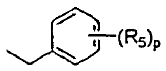 ist;
R₄ Wasserstoff, Alkalimetallion oder C₁-C₇-Alkyl oder Aryl ist;
R₅ unabhängig C₁-C₄-Alkyl, C₁-C₄-Alkyl, substituiert mit wenigstens einem Halogen, oder Halogen ist;
m eine ganze Zahl von 0 bis 4 ist;
n eine ganze Zahl von 0 bis 5 ist und
p eine ganze Zahl von 0 bis 5 ist.

2. Organoselenverbindung gemäß Anspruch 1, die eine racemische Verbindung oder ein optisches Isomer, dargestellt durch die untenstehende Formel III, ist: 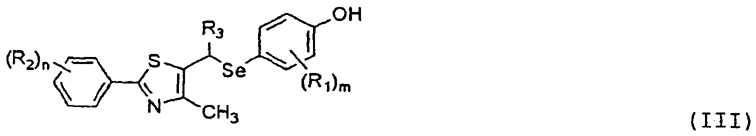 worin R₁ bis R₃, R₅, m, n und p wie in Formel 1 definiert sind.

3. Organoselenverbindung gemäß Anspruch 1, die eine racemische Verbindung oder ein optisches Isomer, dargestellt durch die folgende Formel IV, ist: 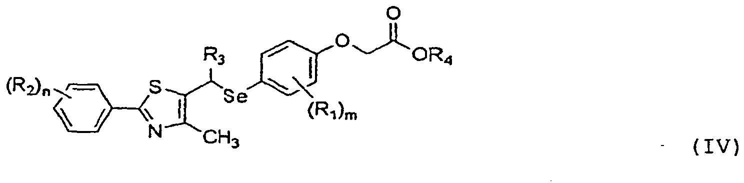 worin R₁ bis R₅, m, n und p wie in Formel 1 definiert sind.

4. Organoselenverbindung gemäß Anspruch 1, die eine racemische Verbindung oder ein optisches Isomer, dargestellt duch die untenstehende Formel IVa, ist: 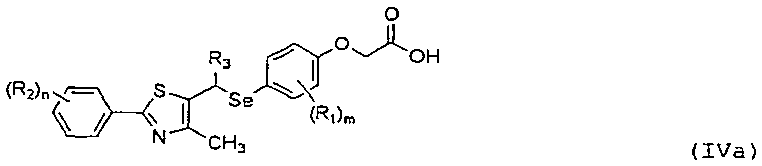 worin R₁ bis R₃, R₅, m, n und p wie in Formel 1 definiert sind.

5. Organoselenverbindung gemäß Anspruch 5, wobei:
R₁ unabhängig Methyl oder Ethyl ist;
R₂ unabhängig Methyl, Ethyl, CF₃, F oder Cl ist;
R₃ Wasserstoff oder 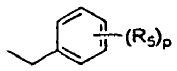 ist;
R₅ unabhängig CF₃, F oder Cl ist;
m eine ganze Zahl von 0 bis 2 ist;
n eine ganze Zahl von 0 bis 3 ist und
p eine ganze Zahl von 0 bis 3 ist.

6. Herstellungsverfahren zur Herstellung der Organoselenverbindung gemäß Anspruch 1, umfassend die Schritte:
a) Umsetzen der Verbindung, die durch die untenstehende Formel V dargestellt wird, mit einem Grignard-Reagens und Umsetzen mit einem Organometallreagens unter Erhalt der Organometallverbindung, wie durch die untenstehende Formel Vb dargestellt wird;
b) anschließend Umsetzen der Organometallverbindung, die durch die Formel Vb dargestellt wird, mit Selen (Se) unter Erhalt der Metall-Selen-Verbindung, die durch die untenstehende Formel IIa dargestellt wird, und
c) anschließendes Umsetzen der Selenverbindung, die durch die Formel IIa dargestellt wird, mit der Thiazolverbindung, die durch die untenstehende Formel VI dargestellt wird, unter Erhalt der Verbindung, die durch die untenstehende Formel IIIa dargestellt wird: 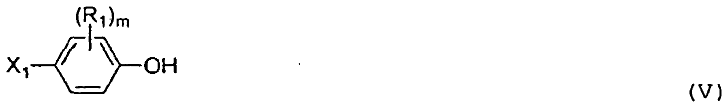 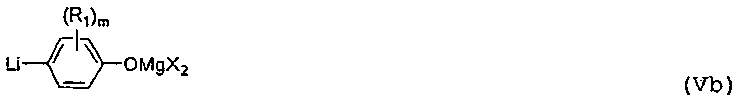 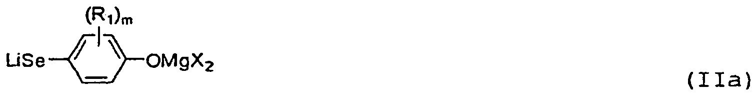 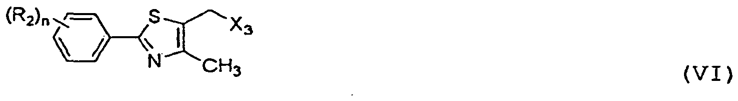 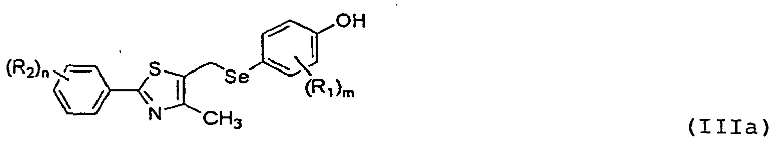
worin X₁ Brom oder Iod ist, X₂ Chlor, Brom oder Iod ist, X₃ Chlor, Brom, Iod oder eine andere Abgangsgruppe, die gute Reaktivität für eine nukleophile Substitution hat, ist und andere Substituenten wie in Formel 1 definiert sind.

7. Herstellungsverfahren gemäß Anspruch 6, das außerdem die Schritte
e) Umsetzen der Verbindung, die durch die untenstehende Formel IIIa dargestellt wird, mit einem Alkylhalogenacetat unter Erhalt der Esterverbindung, die durch die untenstehende Formel IVb dargestellt wird, und
f) Hydrolysieren der Esterverbindung, die durch die Formel IVb dargestellt wird, unter Erhalt der Verbindung, die durch die untenstehende Formel IVc dargestellt wird: 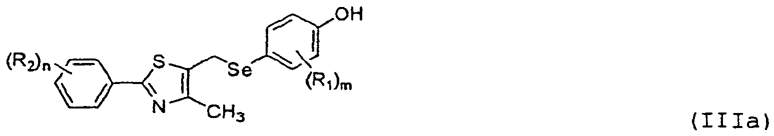 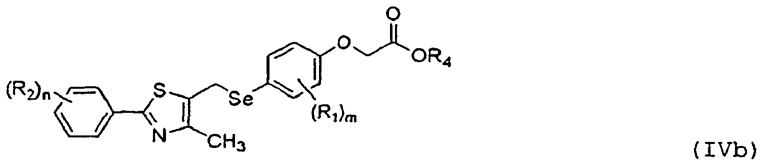 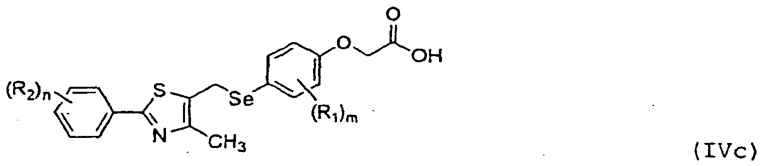
worin R₁ bis R₃, m, n und p wie in Formel 1 definiert sind und R₄ C₁-C₇-Alkyl oder Aryl ist,
umfasst.

8. Herstellungsverfahren gemäß Anspruch 6, das außerdem die Schritte:
g) Schützen der Hydroxylgruppe der durch die untenstehende Formel IIIa dargestellten Verbindung, Umsetzen der Verbindung mit der Verbindung, die durch die untenstehende Formel VII dargestellt wird, unter basischen Bedingungen und Entfernen der Hydroxyl-Schutzgruppe unter Erhalt der Verbindung, die durch die untenstehende Formel IIIb dargestellt wird;
h) Umsetzen der Verbindung, die durch die Formel IIIb dargestellt wird, mit einem Alkylhalogenacetat unter Erhalt der Esterverbindung, die durch die untenstehende Formel IVd dargestellt wird, und
i) Hydrolysieren der durch die Formel IVd dargestellten Esterverbindung unter Erhalt der durch die untenstehende Formel IVe dargestellten Verbindung: 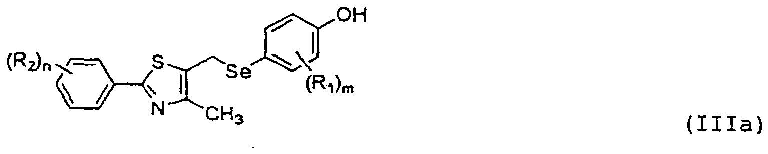 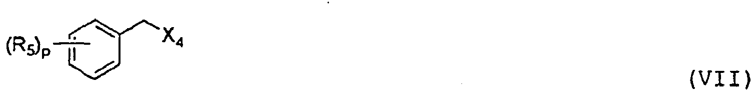 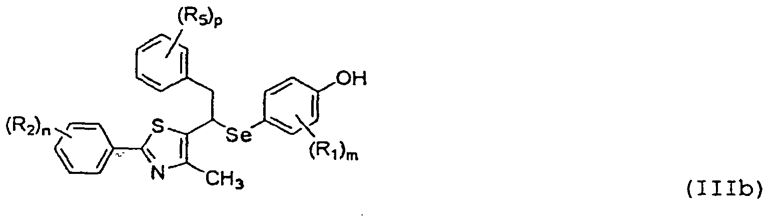 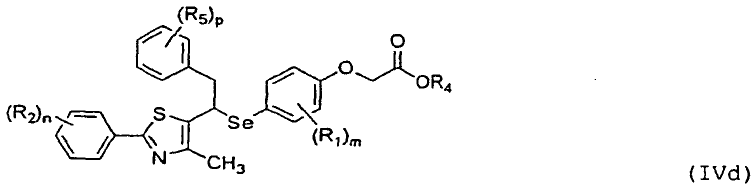 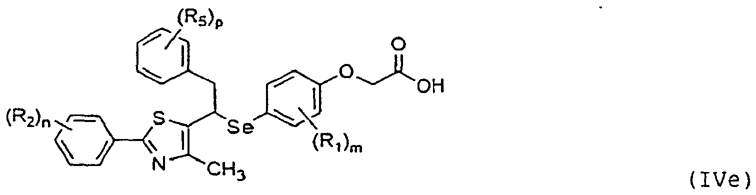
worin R₁ bis R₃, R₅, m, n und p wie in der Formel 1 definiert sind, R₄ C₁-C₇-Alkyl oder Aryl ist und X₄ Chlor, Brom, Iod oder eine andere Abgangsgruppe mit guter Reaktivität für eine nukleophile Substitution ist,
umfasst.

9. Medizinische Zusammensetzung zur Verwendung bei der Behandlung von kardiovaskulären Erkrankungen, Diabetes oder Adipositas oder zur Verwendung als Cholesterin senkendes Mittel, umfassend die Selen enthaltende Verbindung, die durch die Formel IV gemäß Anspruch 3 dargestellt wird, oder ein pharmazeutisch verfügbares Salz der Verbindung als aktives Ingrediens.

10. Nahrungsergänzungsmittel, Gesundheitsgetränk, Nahrungsmittelingrediens oder funktionelles Kosmetikum, das die Selen enthaltende Verbindung, die durch die Formel IV von Anspruch 3 dargestellt wird, oder ein pharmazeutisch verfügbares Salz der Verbindung als aktives Ingrediens umfasst.

11. Peroxisome Proliferator-Activated Receptor δ (PPARδ)-Aktivatorzusammensetzung, die die Selen enthaltende Verbindung, die durch die Formel IV gemäß Anspruch 3 dargestellt wird, oder ein pharmazeutisch verfügbares Salz der Verbindung als aktives Ingrediens umfasst.

## Revendications

1. Composé d'organosélénium représenté par la formule I ci-dessous: 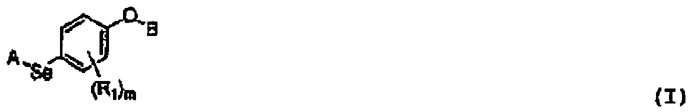 où
A représente 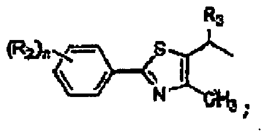 B représente un atome d'hydrogène ou 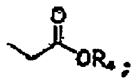 R₁ représente indépendamment un groupe alkyle en C₁ à C₄, alkyloxy en C₁ à C₄, alkylthioxy en C₁ à C₄, alkylamine en C₁ à C₄, un atome de fluor ou de chlore ;
R₂ représente indépendamment un groupe alkyle en C₁ à C₄, alkyle en C₁ à C₄, substitué par au moins un atome d'halogène, ou un atome d'halogène ;
R₃ représente un atome d'hydrogène, un groupe alkyle en C₁ à C₄ ou 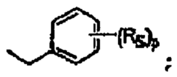 R₄ représente un atome d'hydrogène, un ion de métal alcalin ou un groupe alkyle en C₁ à C₇ ou aryle ;
R₅ représente indépendamment un groupe alkyle en C₁ à C₄, un groupe alkyle en C₁ à C₄ substitué par au moins un atome d'halogène, ou un atome d'halogène ;
m est un nombre entier de 0 à 4 ;
n est un nombre entier de 0 à 5 ; et
p est un nombre entier de 0 à 5.

2. Composé d'organosélénium selon la revendication 1, qui est un composé racémique ou un isomère optique représenté par la formule III ci-dessous : 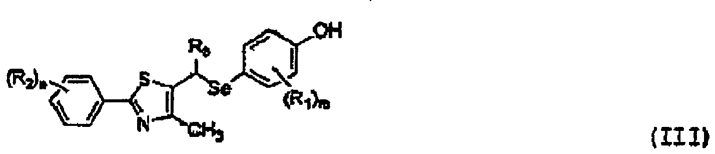 où R₁ à R₃, R₅, m, n et p sont tels que définis dans la formule I.

3. Composé d'organosélénium selon la revendication 1, qui est un composé racémique ou un isomère optique représenté par la formule IV ci-dessous : 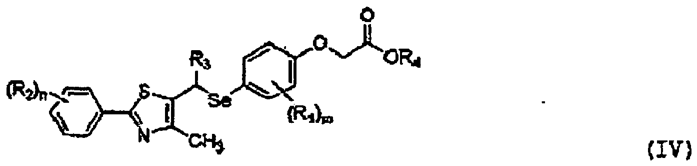 où R₁ à R₅, m, n et p sont tels que définis dans la formule I.

4. Composé d'organosélénium selon la revendication 1, qui est un composé racémique ou un isomère optique représenté par la formule IVa ci-dessous : 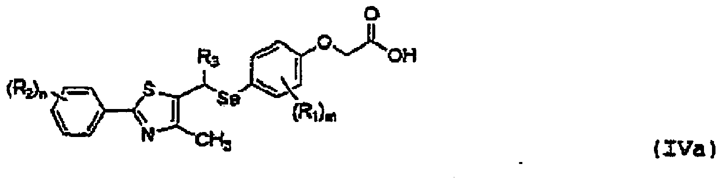 où R₁ à R₃, R₅, m, n et p sont tels que définis dans la formule I.

5. Composé d'organosélénium selon la revendication 1, où :
R₁ représente indépendamment un groupe méthyle ou éthyle ;
R₂ représente indépendamment un groupe méthyle, éthyle, CF₃, F ou Cl ;
R₃ représente un atome d'hydrogène ou 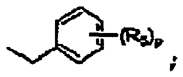
R₅ représente indépendamment CF₃, F ou Cl ;
m est un nombre entier de 0 à 2 ;
n est un nombre entier de 0 à 3 ; et
p est un nombre entier de 0 à 3.

6. Procédé de préparation du composé d'organosélénium selon la revendication 1, comprenant les étapes suivantes :
a) la réaction du composé représenté par la formule V ci-dessous avec un réactif de Grignard et la réaction de ce mélange avec un réactif organométallique pour obtenir le composé organométallique représenté par la formule Vb ci-dessous ;
b) la réaction subséquente du composé organométallique représenté par la formule Vb avec du sélénium (Se) pour obtenir le composé de métal-sélénium représenté par la formule IIa ci-dessous ; et
c) la réaction subséquente du composé de sélénium représenté par la formule IIa avec le composé de thiazole représenté par la formule VI ci-dessous pour obtenir le composé représenté par la formule IIIa ci-dessous : 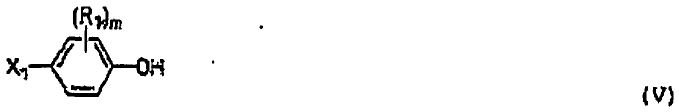 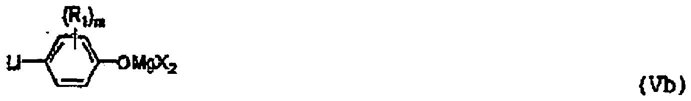 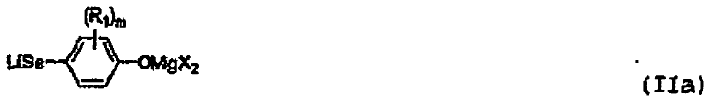 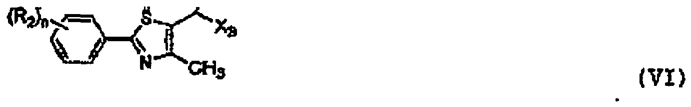 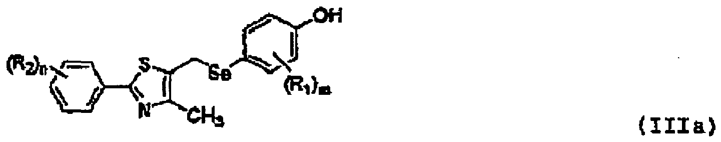
où X₁ représente un atome de brome ou d'iode, X₂ représente un atome de chlore, de brome ou d'iode, X₃ représente un atome de chlore, de brome ou d'iode ou un autre groupe libérable présentant une bonne réactivité pour une substitution nucléophile et les autres substituants sont tels que définis dans la formule I.

7. Procédé de préparation selon la revendication 6, qui comprend en outre les étapes suivantes :
e) la réaction du composé représenté par la formule IIIa ci-dessous avec un halogéno-acétate d'alkyle pour obtenir le composé ester représenté par la formule IVb ci-dessous ; et
f) l'hydrolyse du composé ester représenté par la formule IVb pour obtenir le composé représenté par la formule IVc ci-dessous . 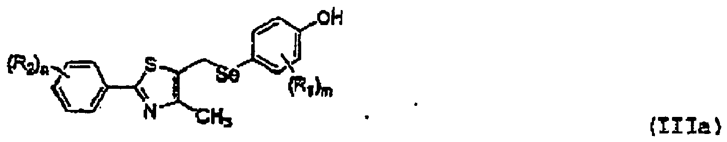 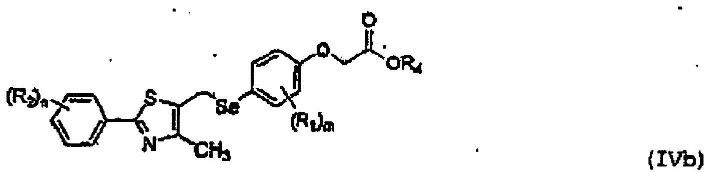 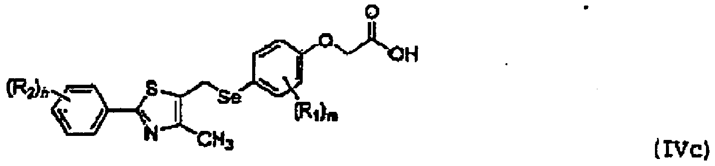
où R₁ à R₃, m, n et p sont tels que définis dans la formule I et R₄ représente un groupe alkyle en C₁ à C₇ ou aryle.

8. Procédé de préparation selon la revendication 6, qui comprend en outre les étapes suivantes :
g) la protection du groupe hydroxyle du composé représenté par la formule IIIa ci-dessous, la réaction du composé avec le composé représenté par la formule VII ci-dessous dans des conditions basiques et l'élimination du groupe hydroxyle-protecteur pour obtenir le composé représenté par la formule IIIb ci-dessous ;
h) la réaction du composé représenté par la formule IIIb avec un halogéno-acétate d'alkyle pour obtenir le composé ester représenté par la formule IVd ci-dessous ; et
i) l'hydrolyse du composé ester représenté par la formule IVd pour obtenir le composé représenté par la formule IVe ci-dessous : 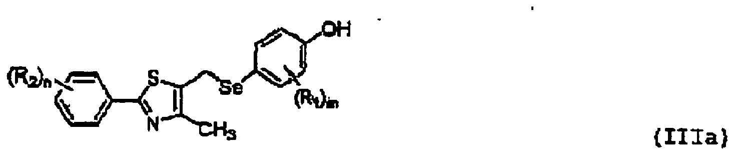 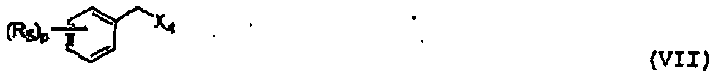 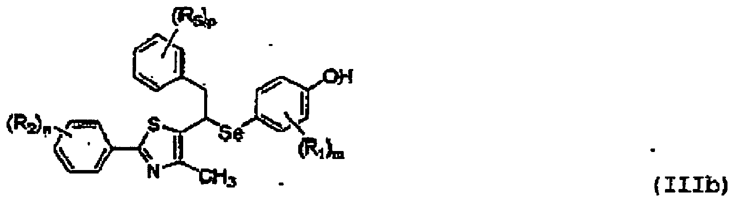 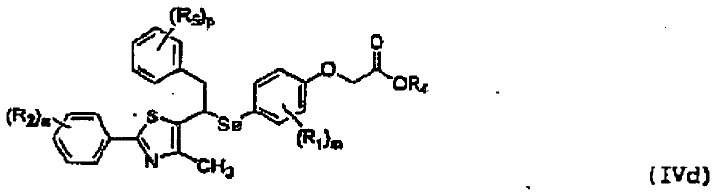 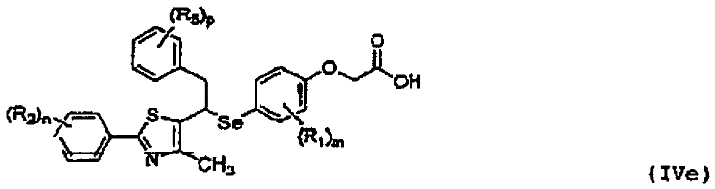
où R₁ à R₃, R₅, m, n et p sont tels que définis dans la formule I, R₄ représente un groupe alkyle en C₁ à C₇ ou aryle et X₄ représente un atome de chlore, de brome, d'iode ou un autre groupe libérable présentant une bonne réactivité pour une substitution nucléophile.

9. Composition médicale pour une utilisation dans le traitement de maladies cardiovasculaires, du diabète ou de l'obésité, ou pour une utilisation en tant qu'hypocholestérolémiant comprenant le composé contenant du sélénium représenté par la formule IV selon la revendication 3 ou un sel pharmaceutiquement disponible du composé en tant que principe actif.

10. Complément alimentaire pour la santé, boisson pour la santé, composant alimentaire ou produit cosmétique fonctionnel comprenant le composé contenant du sélénium représenté par la formule IV selon la revendication 3 ou un sel pharmaceutiquement disponible du composé en tant que principe actif.

11. Composition d'activateur des récepteurs d'activation et de la prolifération des peroxysomes δ (PPARδ) comprenant le composé contenant du sélénium représenté par la formule IV selon la revendication 3 ou un sel pharmaceutiquement disponible du composé en tant que principe actif.
